# EUROPEAN PATENT SPECIFICATION

(11) **EP 2 376 052 B1**
(45) Date of publication and mention of the grant of the patent: **11.10.2017**
(21) Application number: 09796305.2
(22) Date of filing: 14.12.2009
(51) Int. Cl.: A61K 8/26, A61K 8/19, A61K 8/04, A61Q 15/00

(54) **INORGANIC GEL AND COMPOSITION AND PROCESS FOR THE PRODUCTION THEREOF**
ANORGANISCHES GEL UND ZUSAMMENSETZUNG SOWIE HERSTELLUNGSVERFAHREN DAFÜR
GEL ET COMPOSITION INORGANIQUES ET LEUR PROCÉDÉ DE FABRICATION

(30) Priority: 15.12.2008 US 122469 P
(43) Date of publication of application: 19.10.2011
(73) Proprietor: Beiersdorf AG, 20253 Hamburg (DE)
(72) Inventor: MIERTSCH, Heike, 22459 Hamburg (DE); BIEL, Stefan, 21077 Hamburg (DE); WEINERT, Katrin, 22763 Hamburg (DE); NÜBEL, Thomas, 25469 Halstenbek (DE); MAURER, Peter, 24536 Neumünster (DE)
(86) International application number: PCT/EP2009/008924
(87) International publication number: WO 2010/078917

(56) References cited:
- WO-A1-2005/105026
- GB-A- 2 220 568
- US-A- 5 376 363
- US-A1- 2007 003 499
- DATABASE GNPD [Online] MINTEL; 30 November 2004 (2004-11-30), "Antiperspirant Geeli Jaloille", XP002725784, retrieved from Mintel accession no. 319441

## Description

### CROSS-REFERENCE TO RELATED APPLICATIONS

The present application claims priority of U.S. Provisional Application No. 61/122,469, filed December 15, 2008.

### BACKGROUND OF THE INVENTION

### 1. Field of the Invention

The present invention relates to preferably transparent aluminium containing gels, compositions comprising the gels and processes for the production thereof.

### 2. Discussion of Background Information

A large variety of deodorant and antiperspirant (AP) products is commercially available. However, none of these products is completely free from disadvantages. In particular, especially for aesthetic reasons transparent and translucent products are preferred by many consumers. These products are usually of one of three types, i.e., aqueous-alcoholic formulations, water-in-silicone emulsions and microemulsions.

The aqueous-alcoholic deodorant and AP formulations are mostly based on water and alcohol as medium, deodorant and antiperspirant agents as active ingredients, and also perfume, solubilizers and thickeners (mostly based on carbohydrates) as additional agents. They are perceived by the consumer as being fresh and cooling, but are at the same time encumbered with a whole series of disadvantages. For example, application primarily to freshly shaved skin is associated with incompatibilities as a result of the alcohol content. Another disadvantage is the fact that relatively large amounts of oil cannot be incorporated into such systems. As a result of the relatively high amount of antiperspirant salt required for highly effective performance, a white residue remains following application to the skin; this is perceived by the consumer as being inconvenient. However, due to the absence of a sufficiently large oil phase for technical reasons, this residue cannot be concealed. Moreover, the use of carbohydrate thickeners frequently results in a high stickiness of the product after the alcohol has evaporated.

Water-in-silicone emulsions belong to the group of water-in-oil emulsions. The water phase comprising ethanol or polyhydric alcohols, such as, for example, propylene glycol and water-soluble active ingredients, such as AP agent and/or deodorant active ingredient, usually constitutes about 75-90% of the formulation. The oil phase contains a volatile and a nonvolatile silicone oil and also a silicone emulsifier. The transparency of water-in-silicone emulsions is based on matching refractive indices of the two phases. It is a drawback that even a difference in the refractive indices of 0.0004 caused, for example, by evaporation, leads to cloudiness.

One approach of solving the described disadvantages has been made possible through cosmetically pleasing alcohol-free and transparent products which are based on so-called microemulsions. These have the advantage that even relatively large amounts of various oils - with all of the described positive effects for the consumer - can be stably incorporated. Formulations of this type are available primarily by means of phase inversion temperature technology (PIT) or high-pressure homogenization. The required stability of the emulsifier system in the case of high concentrations of antiperspirant salts, however, places high demands on the formulation skill of the product developer. Moreover, drawbacks of these formulations include a sticky feel on the skin caused by the thickener, and the lack of a yield point.

One of the objects of the present invention is to provide a product with antiperspirant activity which is free from at least some of the disadvantages of the known products. Other objects of the present invention will become apparent from the following discussion of preferred aspects thereof. Patent document WO 2005/105026 discloses transparent cosmetic or dermatological formulations.

### SUMMARY OF THE INVENTION

The present invention provides an inorganic gel (hydrogel) which is based on one or more (preferably inorganic) aluminum compounds, one or more compounds selected from inorganic carbonate compounds and inorganic bicarbonate compounds, and water.

In one aspect, the gel of the present invention may be obtainable by a process which comprises contacting (a) one or more (preferably inorganic) aluminum compounds and (b) one or more compounds selected from inorganic carbonate compounds and inorganic bicarbonate compounds in the presence of (c) water in weight ratios of (a), (b) and (c) which result in the formation of a gel.

In another aspect, the gel of the present invention may be a gel which is suitable for cosmetic purposes and/or a gel which has a pH of from about 4 to about 6 and/or a gel which is transparent (or at least translucent).

In another aspect of the gel, (a) may comprise at least one aluminum chloro compound and/or (a) may comprise at least one alum compound.

In another aspect of the gel of the present invention, (a) may comprise at least one compound which has antiperspirant activity and/or forms oligomeric or polymeric species in aqueous solution. For example, (a) may comprise one or more of an aluminum chlorohydrate, an activated aluminum chlorohydrate, aluminum sesquichlorohydrate, and an aluminum zirconium chloro compound, and may particularly comprise an aluminum chlorohydrate (e.g., an aluminum chlorohydrate wherein an atomic ratio Al:Cl is from about 1.5:1 to about 2.5:1).

In yet another aspect of the gel of the present invention, (b) does comprise at least one compound selected from carbonates and bicarbonates of alkali metals (such as, e.g., Na and K), alkaline earth metals (such as, e.g., Mg and Ca), zinc, and

In another aspect, (b) may comprise a carbonate compound and/or may comprise one or more of Na₂CO₃, K₂CO₃, MgCO₃, CaCO₃, NaHCO₃, and KHCO₃, and may particularly comprise one or more of Na₂CO₃, MgCO₃, and CaCO₃, and preferably at least MgCO₃.

In another aspect of the gel of the present invention, the weight ratio (a) : (b) may be from about 20 : 1 to about 2 : 1, e.g., from about 15 : 1 to about 5 : 1, or from about 12 : 1 to about 8 : 1.

The gel of the present invention, (a) may be employed in a concentration of from about 1 % to about 50 % by weight, e.g., a concentration of from about 5 % to about 15 % by weight, a concentration of from about 7 % to about 13 % by weight, or a concentration of from about 9 % to about 11 % by weight, based on the total weight of (a) plus (b) plus (c).

In another aspect of the gel, (b) may be employed in a concentration of from about 0.2 % to about 5 % by weight, e.g., a concentration of from about 0.8 % to about 1.5 % by weight, or a concentration of from about 0.9 % to about 1.2 % by weight, based on the total weight of (a) plus (b) plus (c).

In yet another aspect of the gel, (c) may be employed in a concentration of at least about 70 % by weight, based on the total weight of (a) plus (b) plus (c).

In another aspect of the gel of the present invention, the contacting may take place at a temperature of from about 10°C to about 60°C (e.g., at a temperature of from about 20°C to about 40°C) and/or at about atmospheric pressure.

In yet another aspect, the contacting of (a) and (b) and (c) may take place in the further presence of (d) a perfume (e.g., a perfume which is suitable for cosmetic products) and preferably also a solubilizer for the perfume. For example, the solubilizer may comprise one or more of a polyethoxylated fatty acid mono, di- or triglyceride and a polyethoxylated fatty alcohol and/or the perfume may be present in a concentration of from about 0.2 % to about 2 % by weight and/or the solubilizer may be present in a concentration of from about 1 % to about 10 % by weight, each based on the total weight of (a), (b), (c) and (d).

In another aspect, the gel of the present invention may exhibit a yield point.

In yet another aspect, the gel may further comprise at least one dye and/or a plurality of particles (for example, colored particles) which are (stably) suspended in the gel. For example, the particles may comprise particles which are visible with the unaided eye.

In a still further aspect, the gel of the present invention may have a distinct yield point. The gel may also be present as a substantially solid substance.

In another aspect, the gel of the present invention may be (substantially) free of organic gel formers (and in particular, purely organic gel formers) and/or may be completely inorganic, i.e., (substantially) free of any organic substances and/or residues.

The present invention also provides a (substantially) transparent cosmetic (hydro)gel which exhibits antiperspirant activity. The gel has a pH of from about 4 to about 6 and is obtainable by a process which comprises contacting, at atmospheric pressure and at a temperature of from about 10°C to about 60°C, (a) from about 9 % to about 11 % by weight of at least one of aluminum chlorohydrate and activated aluminum chlorohydrate, and (b) from about 0.9 % to about 1.2 % by weight of at least one of Na₂CO₃, K₂CO₃, MgCO₃, CaCO₃, NaHCO₃, and KHCO₃ in the presence of (c) at least about 70 % by weight of water. All weight percentages are based on the total weight of (a) plus (b) plus (c).

In one aspect, the gel may have a pH of from about 4.5 to about 6.0.

In another aspect, at least about 85 % by weight (e.g., at least about 90 % by weight or at least about 95 % by weight) of all starting materials for the production of the hydrogel may consist of (a), (b) and (c).

In yet another aspect, (a), (b) and (c) may be contacted in the presence of a perfume and a solubilizer for the perfume.

The present invention also provides a gel as set forth above in dried form (i.e., as a solid) which preferably has a water content of not more than about 2 % by weight, e.g., not more than about 1 % by weight.

The present invention also provides a cosmetic (or dermatological) composition which comprises a gel according to the present invention as set forth above (including the various aspects thereof).

In one aspect, the composition may further comprise at least one deodorant active ingredient and/or at least one hydroxycarboxylic acid such as, e.g., mandelic acid.

In another aspect, the composition may be present in a form which is suitable for the topical application to skin. For example, the composition may be present as a gel, a lotion, a cream, or a stick.

In another aspect, the composition (and the gel comprised therein) may be unsuitable for ingestion by a human or animal.

The present invention also provides a glass which is obtainable (e.g., by heating) from the gel of the present invention as set forth above and in particular, from a gel which is purely inorganic, i.e., (substantially) free or organic substances and/or organic residues.

The present invention also provides a method of reducing or preventing perspiration. The method comprises the application of a gel or a cosmetic composition according to the present invention as set forth above (including the various aspects thereof) to skin.

The present invention also provides a method of physical examination by means of ultrasonic radiation. The method comprises applying to skin the gel of the present invention as set forth above (including the various aspects thereof).

The present invention also provides a method of transporting a drug onto or through skin. The method comprises applying to skin the gel of the present invention as set forth above (including the various aspects thereof).

The present invention also provides a process for making a cosmetic aluminum containing gel as well as the gel obtainable thereby. The process comprises contacting (a) one or more (preferably inorganic) aluminum compounds and (b) one or more compounds selected from inorganic carbonate compounds and inorganic bicarbonate compounds in the presence of (c) water in weight ratios of (a), (b) and (c) and under conditions which result in a formation of a gel.

In one aspect, the process may comprise combining (a) with a mixture which comprises (b) and at least a part of (c).

In another aspect, the combining may take place at a temperature of from about 10°C about 60°C.

In yet another aspect of the process of the present invention, the resultant mixture may be agitated at least until the mixture is substantially free from solids and the gel has started to form.

In a still further aspect, the process may further comprise allowing the gel which has formed to be in contact with the atmosphere.

The present invention also provides a preferably transparent (or at least translucent) container which comprises a preferably transparent (or at least translucent) gel and/or composition of the present invention as set forth above (including the various aspects thereof).

In one aspect, the container may comprise one or more organic polymers. In yet another aspect, the walls of the container may have a refractive index which is about the same as (e.g., differs by not more than about 0.0002 units, e.g., not more than about 0.0001 units from) the refractive index of the gel or composition. In a still further aspect, the container may have a color which is different from the color of the gel or composition. In another aspect, the container may be present as a stick dispenser, a gel dispenser, a tube, or a roll-on.

The present invention also provides a method of stabilizing particles which are suspended in a gel (maintaining the particles in the suspended state). The method comprises employing as the gel the gel of the present invention as set forth above (including the various aspects thereof).

The present invention also provides a cosmetic composition which comprises a mixture of (a) one or more inorganic aluminum compounds, and (b) one or more compounds selected from inorganic carbonate compounds and inorganic bicarbonate compounds. The composition comprises not more than about 5 % by weight of water, based on a total weight of the composition.

In one aspect, the composition may exhibit antiperspirant activity when applied to skin.

In another aspect, the composition may be present as a solid.

In yet another aspect, the composition may comprise not more than about 2 % by weight of water.

In a still further aspect, the composition may be capable of forming a gel when contacted with water.

In another aspect, component (a) of the composition may comprise at least one aluminum chloro compound and/or it may comprise at least one alum compound.

In another aspect, component (a) of the composition may comprise at least one compound which has antiperspirant activity and/or forms oligomeric or polymeric species in aqueous solution and/or may comprise at least one of aluminum chlorohydrate, activated aluminum chlorohydrate, aluminum sesquichlorohydrate, and an aluminum zirconium chloro compound and, in particular, aluminum chlorohydrate.

In yet another aspect, component (b) of the composition may comprise at least one carbonate or bicarbonate of an alkali or alkaline earth metal and/or of Zn and/or of ammonium. For example, the alkali or alkaline earth metal may be selected from Na, K, Mg, and Ca and/or component (b) may comprise a carbonate compound and/or may comprise at least one of Na₂CO₃, K₂CO₃, MgCO₃, CaCO₃, NaHCO₃, and KHCO₃.

In another aspect of the composition, the weight ratio (a) : (b) may be from about 20 : 1 to about 2 : 1, e.g., from about 15 : 1 to about 5 : 1, or from about 12 : 1 to about 8 : 1.

The present invention also provides a method of reducing or preventing perspiration and a method of in situ preparation of an anti-transpirant gel. These methods comprise applying to skin the composition of the present invention as set forth above (including the various aspects thereof).

### BRIEF DESCRIPTION OF THE DRAWINGS

The present invention is further described in the detailed description which follows, in reference to the noted plurality of drawings by way of non-limiting examples of exemplary embodiments of the present invention, in which like reference numerals represent similar parts throughout the several views of the drawings, and wherein:
Fig. 1 is a graph which shows the viscosity of the gel of Example 1 with one type of perfume incorporated therein as a function of the shear stress; and
Fig. 2 is a graph which shows the viscosity of the gel of Example 1 with another type of perfume incorporated therein as a function of the shear stress.

### DETAILED DESCRIPTION OF THE INVENTION

The particulars shown herein are by way of example and for purposes of illustrative discussion of the embodiments of the present invention only and are presented in the cause of providing what is believed to be the most useful and readily understood description of the principles and conceptual aspects of the present invention. In this regard, no attempt is made to show details of the present invention in more detail than is necessary for the fundamental understanding of the present invention, the description and drawings making apparent to those skilled in the art how the several forms of the present invention may be embodied in practice.

The gel of the present invention is obtainable, for example, by combining at least two starting materials, i.e., at least one (preferably purely inorganic) aluminum compound (preferably selected from aluminum chloro compounds and alum compounds) and at least one inorganic carbonate or bicarbonate compound in the presence of water. Of course, there are several optional materials which may be employed in addition to these starting materials, some of which will be discussed in some detail below.

Typical examples of aluminum compounds which are suitable for making the gel of the present invention include compounds which exhibit antiperspirant activity and/or do not completely dissociate in water and form oligomeric/polymeric species in water. For example, the term "aluminum chloro compound" as used herein and in the appended claims preferably does not encompass a chloride compound such as AlCl₃, although AlCl₃ may be employed, either alone or in combination with one or more other aluminum compounds (although this is not preferred). If AlCl₃ is employed, it preferably accounts for not more than about 50 mole-%, e.g., not more than about 25 mole-%, or not more than about 10 mole-% of all compounds (a) that are used for making the gel of the present invention.

Non-limiting examples of aluminum compounds which are suitable for use, alone or as a combination of two or more thereof, in the production of the gel of the present invention include compounds of the general formula Al₂(OH)₆₋ₐXₐ wherein X is Cl, Br, I or NO₃ (preferably Cl) and a is from about 0.3 to about 6, preferably from about 0.8 to about 2.5, more preferably from about 1 to about 2 (such that the atomic ratio A1 : X is from about 0.9:1 to about 2.1:1). These compounds generally contain some water of hydration, typically of the order of 1 to 6 moles per mole of compound. More preferably, the aluminum compounds comprise compounds of the above formula wherein X represents Cl and wherein the atomic ratio Al:Cl is at least about 1:1, e.g., at least about 1.5, at least about 1.7:1, or at least about 1.9:1, and not higher than about 2.5:1, e.g., not higher than about 2.3:1 or not higher than about 2.1: 1. Non-limiting specific examples of aluminum (chloro) compounds for use in the present invention include aluminum salts of the empirical formula [Al₂(OH)ₘClₙ] (with m+n = 6) such as, e.g.,
- aluminum chlorohydrate [Al₂(OH)₅Cl] x H₂O standard aluminium complexes: Locron L, Locron LIC, Locron LIF (Clariant), Chlorhydrol (Reheis), ACH-303 (Summit), Aloxicoll L (Guilini) activated aluminum complexes: Reach 501 (Reheis), Aloxicoll 51L
- aluminum sesquichlorohydrate [Al₂(OH)₄.₅Cl_{1.5}] x H₂O standard aluminium complexes: Aloxicoll 31L (Guilini), Westchlor 186 (Westwood Chemicals) activated aluminum complexes: Reach 301 (Reheis)
- aluminum dichlorohydrate [Al₂(OH)₄Cl₂] x H₂O.

Regarding the activated aluminum chlorohydrate which is an example of a suitable aluminum starting material for making the gel of the present invention, it is noted that this term represents aluminum chloro compounds which are present in the form of oligomeric and/or polymeric species. The preferred activated species has been theorized as having the structure {AlO₄ Al₁₂ (OH)₂₄ (H₂O)₁₂}₇ and is sometimes referred to as Al₁₃, Band III or a "peak 3" material. The "peak 3" terminology comes from the fact that it is typically the 3rd peak or 3rd material to be eluted when using High Performance Liquid Chromotography, HPLC, to characterize aluminum chlorohydrate salts (according to the method taught in EP 0256831). Regular salts are typically those that have more of "peak 2" material (theorized as being solublized Al(OH)₃ entities held together by H bonding and adjunct Cl ions) than "peak 3" material.

Of course, aluminum chloro compounds which are different from those set forth above may be employed as well, although their use may be less advantageous, especially when a (substantially) transparent (clear) gel with a defined yield point or a substantially solid gel is to be obtained. Non-limiting examples of such further aluminum chloro compounds include aluminum-zirconium chloro compounds such as, e.g., those of formulae [Al₄Zr(OH)₁₃Cl₃] x H₂O x Gly (Gly = glycine), [Al₄Zr(OH)₁₂Cl₄] x H₂O x Gly, [Al₈Zr(OH)₂₃Cl₅] x H₂O x Gly, [Al₈Zr(OH)₂₀Cl₈] x H₂O x Gly, and activated and/or glycine-free forms of these compounds.

Alum compounds for use as starting materials for making the gel of the present invention include all compounds of the general formula AB(SO₄)₂ x 12 H₂O. These compounds are usually present as double sulfates of the general formula A₂SO₄ x B₂(SO₄)₃ x 24 H₂O, wherein A is a monovalent cation (such as, e.g., an alkali metal cation such as Na⁺ and K⁺ or an ammonium cation such as NH₄⁺ and CH₃NH₃⁺) and B represents Al³⁺. A preferred alum compound for use in the present invention is a compound of the above general formula wherein A = K, i.e., KAl(SO₄)₂ x 12 H₂O.

The one or more aluminum compounds will usually be employed in a (total) concentration of at least about 1 %, e.g., at least about 3 %, at least about 5 %, at least about 7 %, or at least about 9 % by weight, and not more than about 50 %, e.g., not more than about 40 %, not more than about 30 %, not more than about 20 %, not more than about 15 %, not more than about 13 %, not more than about 12 %, or not more than about 11 % by weight, based on the total weight of (a) plus (b) plus (c). The above concentrations refer to the so-called active contents of the aluminum compounds. For example, in the case of the aluminum compounds they refer to the water-free compounds and in the case of the aluminum/zirconium compounds they refer to the water- and buffer-free (e.g., glycine-free) compounds.

Non-limiting examples of inorganic carbonate and bicarbonate compounds which are suitable for making the gel of the present invention include the carbonates and bicarbonates of alkali and alkaline earth metals such as, e.g., Li, Na, K, Mg, and Ca. Carbonates are usually preferred. Preferred carbonate compounds include Na₂CO₃, MgCO₃, and CaCO₃. MgCO₃ is particularly preferred. In this regard, it is to be noted that the consistency of the gel usually increases with decreasing radius of the metal ion.

Mixtures of two or more carbonate compounds or of two or more bicarbonate compounds and mixtures of one or more carbonate compounds and one or more bicarbonate compounds may, of course be employed. Further, the one or more (bi)carbonate compounds for use in the present invention may be used not only in pure form but can also be employed in hydrated form and/or in the form of (bi)carbonate containing naturally occurring materials such as, e.g., minerals. Non-limiting examples thereof include natron, trona, potash, magnesite, barringtonite, nesquehonite, lansfordite, artinite, hydromagnestite, dypingite, calcite, vaterite, chalk, limestone, dolomite, and curative chalk of Ruegen ("Ruegener Heilkreide"). Carbonates and bicarbonates (collectively referred to as "(bi)carbonates) which are different from those mentioned above may be used as well, such as, e.g., ammonium (bi)carbonate, zinc (bi)carbonate and manganese (bi)carbonate. Also, the one or more (bi)carbonates may be used in combination with one or more other compounds such as, e.g., oxides, hydrated oxides and hydroxides of alkali and alkaline earth metals, aluminum, zinc, silicon, etc., although there usually is no advantage associated therewith.

Although the particle sizes of the (bi)carbonate compounds for use in the present invention are not critical, compounds having an average particle size (diameter) of from about 0.1 µm to about 100 µm, e.g., from about 1 µm to about 30 µm, may often be advantageous.

The one or more inorganic carbonate and/or bicarbonate compounds will usually be employed in a (total) concentration of at least about 0.2 %, e.g., at least about 0.5 %, at least about 0.7 %, at least about 0.8 %, or at least about 0.9 % by weight, and not more than about 2 %, e.g., not more than about 1.5 %, not more than about 1.4 %, not more than about 1.3 %, not more than about 1.2 %, or not more than about 1.1 % by weight, based on the total weight of (a) plus (b) plus (c). These concentrations refer to the non-hydrated compounds.

The weight ratio (a) : (b) will usually be at least about 2 : 1, e.g., at least about 5 : 1, at least about 7 : 1, at least about 8 : 1, or at least about 9 : 1, and not higher than about 20 : 1, e.g., not higher than about 15 : 1, not higher than about 13 : 1, not higher than about 12 : 1, or not higher than about 11 : 1. Again, these weight ratios are based on the weights of the non-hydrated compounds.

It will be apparent to one of ordinary skill in the art that the most advantageous concentrations and weight ratios of components (a), (b) and (c) depend on various factors such as, e.g., the specific aluminium compound(s) employed, the specific (bi)carbonate compound(s) employed, the desired consistency of the gel, the desired transparency of the gel, optionally employed additional compounds, etc. By way of non-limiting example, in the case of an aluminum chloro compound wherein the atomic ratio of Al:Cl is about 2:1 (hereafter "ACH") and an alkaline earth metal carbonate such as MgCO₃ (hereafter "XCO₃"), a molar ratio XCO₃:ACH of from about 0.10:1 to about 0.35:1 and in particular, from about 0.15:1 to about 0.30:1 will usually result in a gel of satisfactory consistency and transparency.

In this regard, it is noted that in colloid chemistry, the term "gel" denotes a material which is between a solid and a semisolid state and which appears amorphous under a microscope. A gel can also be described as a water-filled three-dimensional network. Typically, gels possess a storage modulus G'(w) which exhibits a pronounced plateau at higher frequencies (on the order of seconds) and a loss modulus G" (w) which is considerably smaller than the storage modulus in the plateau region. Also, merely by way of example, a gel of the present invention may have a viscosity of from about 100 to about 500,000 MPa.s (measured by using a Brookfield RV viscometer with spindle F at 2.5 rpm), or from about 2,000 to about 300,000 MPa.s (measured by using a Brookfield RVT viscometer with spindle D at 2.5 rpm).

The starting materials (a) and (b) for the preparation of the gel (hydrogel) of the present invention will usually be contacted in the presence of water. The water is usually employed in a concentration of at least about 70 % by weight, e.g., at least about 75 % by weight, at least about 80 % by weight, or at least about 85 % by weight, based on the total weight of the one or more aluminum compounds, the one or more inorganic (bi)carbonate compounds and water. These concentrations include the water which may be present in the one or more aluminum compounds and/or the one or more inorganic (bi)carbonate compounds.

Further, a part of the water for use in making the gel of the present invention may be replaced by one or more liquids which are miscible with water. Generally, the total concentration of these one or more liquids is not higher than about 20 %, e.g., not higher than about 15 %, not higher than about 10 %, or not higher than about 5 %, based on the total weight of the resultant gel. Non-limiting examples of corresponding liquids are optionally substituted monohydric and polyhydric alcohols such as, e.g., ethanol, propanol, ethylene glycol, diethylene glycol, propylene glycol, glycerol, butylene glycol, 2-methylpentane-2,4-diol, 2-ethoxyethanol, and 2-butoxyethanol. It is pointed out that these compounds are not necessary for gel formation, but are used, if at all, merely to impart desirable properties (e.g., cooling effect) to the gel of the present invention.

Further non-limiting examples of corresponding liquids include solubilizers for a perfume (fragrance) which may optionally be incorporated into the gel (the perfume will usually be employed in concentrations of from about 0.1 % to about 2 % by weight, preferably from about 0.5 % to about 1.5 % by weight, based on the total weight of the gel).

Preferred examples of solubilizers include polyalkoxylated (e.g., polyethoxylated and/or polypropoxylated) fatty acid mono-, di- and triglycerides and polyalkoxylated (e.g., polyethoxylated and/or polypropoxylated) fatty alcohols, each with a degree of ethoxylation of from about 5 to about 100, e.g., from about 20 to about 80. Particularly advantageous solubilizers include polyethoxylated hydrogenated castor oils such as, in particular, PEG-40 hydrogenated castor oil which is commercially available under various trade names (e.g., Solutor, Eumulgin, Fancol, etc.), PEG-60 hydrogenated castor oil and isosteareth-20.

Solubilizers such as hydrogenated castor oils are advantageous, in particular, in that in their presence perfumes can usually be incorporated into the gel of the present invention in sufficient quantities (e.g., at a concentration of at least about 1 % by weight, based on the total weight of the gel) without rendering the gel opaque. Suitable concentrations of the solubilizer depend on, *inter alia,* the type of the perfume(s) to be incorporated, but concentrations of solubilizer (e.g., polyethoxylated hydrogenated castor oil) of at least about 5 % by weight (and up to about 10 % by weight) relative to the total weight of the resultant gel will usually afford very advantageous results. Another advantage of the above (and other) solubilizers is that in their presence the rate of gel formation can frequently be increased significantly.

The starting materials for making the gel of the present invention can be combined at room temperature, although temperatures which are higher or lower than room temperature are suitable as well. Often the temperature at which the combining takes place will be not lower than about 5°C, e.g., not lower than about 10°C, not lower than about 15°C, or not lower than about 20°C and will be not higher than about 60°C, e.g., not higher than about 50°C, not higher than about 40°C, not higher than about 35°C, or not higher than about 30°C. The contacting pressure will usually be ambient (atmospheric) pressure, although higher and lower pressures may be used as well. When working at room temperature, the formation of the gel will often be substantially complete within about 30 minutes, although in the presence of solubilizers (see above), the time which is required for the substantial completion of the gel formation may be as short as about 2 minutes, or even shorter.

In a preferred embodiment of the process for making a gel according to the present invention the one or more carbonate and/or bicarbonate compounds are suspended and/or dissolved in at least a part of the water and are then combined (preferably with stirring) with the one or more aluminum compounds, either as such or combined with a part of the water. If a solubilizer (and a perfume) is to be employed the aluminum compound(s) may, for example, first be combined with the solubilizer (and the perfume) and then combined with the mixture of (bi)carbonate(s) and water, or the aluminum compound(s) may be combined with the mixture of (bi)carbonate(s), water and solubilizer/perfume. Once the starting materials are combined, stirring will preferably be continued until the formation of the gel starts.

The gel of the present invention is preferably clear, i.e., (substantially) transparent and preferably also (substantially) colorless. As noted above, the concentration ranges of the starting materials within which transparent gels are formed depend predominantly on the starting materials employed and can be determined by routine experimentation. For example, when using only water, 10 % by weight of aluminum chlorohydrate having a ratio of Al:Cl of about 2:1 (calculated as anhydrous compound) and a carbonate, a clear gel will result within the concentration ranges (by weight) of from about 1.0 % to about 1.2 % of CaCO₃, from about 0.9 % to about 1.0 % of MgCO₃ and from about 0.9 % to about 1.1 % by weight of Na₂CO₃. For the corresponding bicarbonates the concentration ranges for forming transparent gels usually are somewhat higher than those for the corresponding carbonates (e.g., about twice as high). A clear gel will also result with water, 1 % by weight of carbonate compound and aluminum chlorohydrate (Al:Cl = about 2:1) in the concentration ranges (by weight of active substance) of from about 9 % to about 10 % (for CaCO₃), from about 9 % to about 13 % (for MgCO₃), and from about 7 % to about 10 % (for Na₂CO₃ as carbonate compound).

In this regard, it is noted that in the present specification and the appended claims the terms "transparent" and "clear" are intended to connote the usual dictionary definition; thus, a transparent gel or composition according to the present invention allows ready viewing of objects behind it. By contrast, a translucent gel or composition, although allowing light to pass through, causes the light to be scattered so that it will be impossible to see clearly objects behind the translucent gel or composition.

For example, within the context of this invention, a gel or composition may be deemed to be transparent or clear if the maximum transmittance of light of any wavelength in the range of from 400 nm to 800 nm (e.g., light having a wavelength of 420 nm) through a sample having a thickness of 1 cm is at least about 40 %, preferably at least about 50 %, or at least about 60 %. The gel or composition is deemed translucent if the maximum transmittance of light is less than about 2 %. The transmittance may be measured by placing a sample of the aforementioned thickness into the light beam of a spectrophotometer whose working range includes the visible spectrum.

The gel according to the present invention will usually have a pH in the as formed state of not lower than about 4.0, e.g., not lower than about 4.2, not lower than about 4.4, not lower than about 4.5 not lower than about 4.7 or not lower than about 5.0, and not higher than about 6.5, e.g., not higher than about 6.3, not higher than about 6.2, not higher than about 6.0, or not higher than about 5.8. Further, the gel preferably is present as a semi-solid with a defined yield point or is present as a virtual solid. Additionally, especially when left standing in contact with the ambient atmosphere (air), the gel may further solidify and become a solid (usable for example, as a stick). The solid may, for example, be comminuted (crushed, milled, etc.) and may in this form be incorporated into cosmetic and other compositions. Conversely, when kept in a hermetically sealed container for extended periods of time the gel may soften (and may even liquefy).

The yield point is a term for the smallest shear stress above which a plastic material behaves in rheological terms like a liquid (DIN 1342-1: 1983-10, incorporated by reference herein). The yield point is determined by recording a flow curve (DIN 53019: 1980-05; DIN 53214: 1982-02, incorporated by reference herein). The value obtained is dependent on the timescale (stress rate) on which the measurement is based. This is independent of whether the measurement is carried out using a shear stress-controlled or speed-controlled viscometer. Short time scales (rapid stresses) generally produce higher values for the yield point. An excessively high yield point may be the cause of flow disturbances. On the other hand, with a suitably adjusted yield point it is possible to suppress the tendency of the gel to run.

Several substances, when incorporated into the gel of the present invention, may improve one or more of the properties thereof. For example, it has been found that low molecular weight organic acids (molecular weight preferably not higher than about 300, e.g., not higher than about 250) and in particular polycarboxylic acids and/or hydroxycarboxylic acids such as citric acid and ascorbic acid and amino acids (preferably having a molecular weight of not higher than about 150, e.g., not higher than about 100) such as alanine and glycine have a positive effect on the flow properties of the gel. For example, when employed in suitable concentrations (e.g., at least about 0.1 % by weight, e.g., at least about 0.3 % by weight, at least about 0.5 % by weight, at least about 0.7 % by weight, but not more than about 5 % by weight, e.g., not more than about 3 % by weight, or not more than about 2 % by weight, each based on the total weight of (a), (b) and (c), and preferably concentrations of from about 1 % to about 10 % by weight in the case of amino acids) these compounds can improve, e.g., the smoothness of the gel, its dispensability from containers (no clogging of nozzles, only small pressure required for discharge from a container such as a tube), and the feeling of the gel on skin.

Further, the gels of the present invention may be (freeze-)stabilized against agglomeration due to being subjected to low temperatures by incorporating therein certain substances such as monohydric and/or polyhydric alcohols (ethanol, propylene glycol, etc.) and polysaccharides such as cellulose and derivatives thereof (e.g., hydroxyethylcellulose). An effective freeze stabilization of the gels of the present invention can usually be achieved with concentrations of from about 2 % to about 10 % by weight in the case of alcohol(s) and from about 0.1 % to about 1 % by weight of polysaccharide(s) and/or derivative(s) thereof, each based on the total weight of (a), (b) and (c)). Of course, combinations of freeze-stabilizers of different types can be used as well.

The gel according to the present invention preferably exhibits antiperspirant acitivity. The antiperspirant activity will often be higher than the antiperspirant activity of a water-in-silicone emulsion made from the same amount of aluminum compound(s).

Due to its relatively high consistency (presence of a yield point), a gel of the present invention will often allow the incorporation of various particles without giving rise to a settling of the particles and/or a mixing thereof with other components which may be present in the gel. The particles can be added before, during and/or after the preparation of the gel, although a uniform incorporation of particles into the finished gel may sometimes be cumbersome.

As used herein and in the appended claims, the term "particles" is meant to include any liquid, solid, semisolid or gaseous matter which is present in particulate form such as, e.g., in the form of (solid or semisolid) grains, beads, flakes, fibers (micro)spheres, liquid drops (comprising, for example, neat liquids, solutions, emulsions, dispersions, etc.) and gas (e.g., air, carbon dioxide, nitrogen, oxygen, etc.) bubbles. The solid or semisolid particles may, for example, be solid particles, hollow (i.e., gas filled) particles (e.g., hollow microspheres) or hollow particles which are completely or partially filled with one or more solid, semisolid or liquid substances (such as, e.g., microcapsules). For example, they may impart one or more of sparkle, pearlescence, color and reflectivity to the preparation.

The particles may be hard, soft, of regular shape (e.g., round or oval), of irregular shape, colored (e.g., blue, red, green, orange, purple, pink and yellow and any mixtures thereof), black or uncolored (white). They may be present in the gel of the present invention in any concentration, preferably in a concentration of at least about 0.001 % by volume, e.g., at least about 0.01 % by volume, at least about 0.1 % by volume, or at least about 1 % by volume, relative to the total volume of the (preferably liquid) preparation. The particle concentration will usually be not higher than about 50 % by volume, e.g., not higher than about 30 % by volume, not higher than about 20 % by volume or not higher than about 10 % by volume of the total volume of the gel.

Also, the particles may comprise any constituents, particularly those which are suitable for use in cosmetic or dermatological preparations. By way of non-limiting example, they may comprise one or more substances selected from preservatives, bactericides, (inorganic and organic) UV filter substances, antioxidants, (preferably water-soluble) vitamins, mineral substances, cosmetically or dermatologically active substances, perfumes, antifoaming agents, dyes, (coloring) pigments, emollients, moisturizers, humectants, deodorizers, opacifiers, binders, buffering agents, chelating agents, viscosity controlling agents, emulsifiers, emulsion stabilizers, film formers, organic solvents (e.g., alcohols, polyols), foam stabilizers, fats, oils, waxes and/or silicone derivatives. Of course, any mixtures of different particles may be present as well, such as, e.g., mixtures of solid and/or semisolid particles and gaseous particles, mixtures of two or more types of solid particles of different form and/or different size and/or different composition and/or different color.

At least some (and preferably substantially all) of the particles will preferably be visible with the unaided eye. In particular, the particles will preferably comprise particles with a size (diameter) of from about 200 nm (e.g., at least about 500 nm, at least about 1 µm, at least about 10 µm, at least about 0.1 mm, or at least about 0.5 mm) up to several millimeters, preferably up to about 10 mm (e.g., up to about 5 mm, up to about 3 mm, up to about 2 mm or up to about 1 mm). Usually, at least about 80 %, e.g., at least about 90 %, at least about 95 %, or at least about 98 % of the particles will have a size within a particular size range, e.g., from about 0.5 mm to about 5 mm.

Non-limiting examples of particles which are suitable for use in the present invention include the gel beads which are described in US 2004/0228886 A1, the entire disclosure whereof is incorporated by reference herein.

Examples of commercially available beads which are suitable for use in the present invention are available under the trade names Cosmospheres GMM-S (made by Pelletech Ltd., Switzerland; diameter 1.1-1.5 mm; green; comprising mannitol, microcrystalline cellulose, CI 77289 (Chromium Hydroxide Green) and lactic acid), Cosmospheres BMM-M (made by Pelletech Ltd., Switzerland; diameter 1.1-1.5 mm; blue; comprising mannitol, microcrystalline cellulose, CI 74160 (Pigment Blue 15) and lactic acid), Beads Cosmo YS-S (made by Pelletech Ltd., Switzerland; diameter 0.5-1.0 mm; yellow; comprising lactose, microcrystalline cellulose, Helianthus Annuus and CI 77492); Unispheres RP-572 (available from Induchem AG, Switzerland; diameter 0.5-0.9 mm; pink; comprising lactose, cellulose, hydroxypropylmethylcellulose, panthenyl triacetate, CI 73360); Unispheres UE-507 (available from Induchem AG, Switzerland; diameter 0.5-0.9 mm; purple; comprising lactose, cellulose, hydroxypropylmethylcellulose, tocopheryl acetate, CI 77007 (Pigment Blue 29); Cosmospheres Beads BCG2-L (made by Pelletech Ltd., Switzerland; diameter 1.5-2.0 mm; blue, glittering; comprising lactose, polyethylene terephthalate, microcrystalline cellulose, Acrylates Copolymer and Pigment Blue 15) and Cosmospheres Beads WCG-G-C-L (made by Pelletech Ltd., Switzerland; diameter 1.5-2.0 mm; gold, glittering; comprising lactose, polyethylene terephthalate, microcrystalline cellulose, shellac, Acrylates Copolymer, mica, titanium dioxide and iron oxides).

If present, the particles are preferably uniformly and stably suspended in the gel of the present invention, i.e., even in the case of a relatively long standing time such as e.g., about a week or even about a month or about a year, the force of gravity does not result in the particles sinking to the bottom to any significant extent.

The appearance of the gel of the present invention or of a (for example, cosmetic or dermatological) composition comprising the gel may also be enhanced by incorporating therein one or more dyes. A large number of different dyes may be used for this purpose. Preferred are dyes which are soluble in water to at least some extent. Non-limiting examples of suitable dyes include methylene blue and dyes with the following Color Index numbers: 15985, 16035, 19140, 42090, 61570, 77491, 77492 and 77499.

The gel of the present invention or a composition comprising the gel may further comprise a (preferably transparent or at least translucent) antiperspirant gel formed from (a) at least one (preferably inorganic) aluminum compound, (b) at least one α-hydroxycarboxylic acid, and (c) water as essential components. The latter gel may have been combined with the gel of the present invention or may have been produced concurrently therewith. Non-limiting examples of component (a) include the aluminum compounds which are suitable for making the gel of the present invention. The term "α-hydroxycarboxylic acid" includes any (preferably aliphatic or araliphatic) organic acid which, besides one or more COOH groups, comprises one or more OH groups in an α-position relative to one of the carboxylic acid functionalities. These acids therefore have the properties of carboxylic acids and alcohols or phenols at the same time. The α-hydroxycarboxylic acids include naturally occurring substances, such as mandelic acid, (o-, p- or m-) hydroxymandelic acid, glycolic acid, lactic acid, malic acid, tartaric acid, citric acid and other fruit acids. Usually, the α-hydroxycarboxylic acids will have from about 2 to about 20 carbon atoms (including the carboxylic group(s)), preferably from about 4 to about 12 carbon atoms. They usually will have 1 to about 3 carboxylic acid groups and 1 to about 3 hydroxy groups. A preferred α-hydroxycarboxylic acid is mandelic acid. Regarding further details with respect to the preparation of gels based on antiperspirant active ingredients, α-hydroxycarboxylic acids and water reference may be made, for example, to WO 2005/105026, the entire disclosure whereof is incorporated by reference herein.

Deodorants are further non-limiting examples of components which may be incorporated into the gels and compositions of the present invention, for example those which are intended for antiperspirant purposes. As is the case with all other optional components of the gels and the cosmetic or dermatological compositions of the present invention, the deodorants can be present in or in the form of suspended particles, if any, and/or in the medium in which the particles are suspended.

Customary cosmetic deodorants are based on various activity principles. By using antimicrobial substances in cosmetic deodorants it is possible to reduce the bacteria flora on the skin. Here, in the ideal case, only the odor-causing microorganisms should be effectively reduced. The flow of perspiration itself is not influenced as a result, in an ideal case only the microbial decomposition of the perspiration is stopped temporarily. The combination of astringents with antimicrobially effective substances in one and the same composition is also customary.

All active ingredients which are customary for deodorants can advantageously be used for the purposes of the present invention, for example, odor concealers, such as customary perfume constituents, odor absorbers, for example the sheet silicates described in DE 40 09 347, of these in particular montmorillonite, kaolinite, illite, beidellite, nontronite, saponite, hectorite, bentonite, smectite, also, for example, zinc salts of ricinoleic acid. Antimicrobial agents may likewise be desirable for use in the present invention. Advantageous substances are, for example, 2,4,4'-trichloro-2'-hydroxydiphenyl ether (Irgasan), 1,6-di(4-chlorophenylbiguanido)hexane (chlorhexidine), 3,4,4'-trichlorocarbanilide, quaternary ammonium compounds, oil of cloves, mint oil, thyme oil, triethyl citrate, farnesol (3,7,11-trimethyl-2,6,10-dodecatrien-1-ol), and the active agents described in DE 37 40 186, DE 39 38 140, DE 42 04 321, DE 42 29 707, DE 42 29 737, DE 42 37 081, DE 43 09 372, DE 43 24 219. The entire disclosures of the above documents are incorporated by reference herein. Further examples of commercially available deodorants include phenethyl acetate, phenethyl alcohol, zinc bisgluconate, zinc dilactate, zinc dipalmitate, zinc diricinoleate and linalool. Sodium hydrogencarbonate can also be used advantageously. The amount of deodorants (one or more compounds), if present, in the compositions of the present invention is preferably from about 0.01% to about 10% by weight, more preferably from about 0.05% to about 5% by weight, based on the total weight of the composition.

The gels of the present invention and (cosmetic and dermatological) compositions comprising them can, of course, comprise a variety of other cosmetic auxiliaries, especially those which are customarily used in cosmetic and dermatological compositions (incorporated into the gel and/or as separate components of the compositions) such as, e.g. preservatives, bactericides, (inorganic and organic) UV filter substances, antioxidants, stabilizers, (preferably water-soluble) vitamins, plant extracts, mineral substances, cosmetically or dermatologically active substances (e.g., coenzyme Q₁₀, creatine and derivatives thereof, carnitine and derivatives thereof, licochalcones, in particular, licochalcone A), antifoaming agents, dyes, (coloring) pigments, insect repellents, tanning agents, self-tanning agents (e.g. dihydroxyacetone), depigmenting agents (e.g. 8-hexadecene-1,16-dicarboxylic acid (dioic acid, CAS Number 20701-68-2; provisional INCI name Octadecenedioic acid)), emollients, moisturizers, humectants, re-fatting agents, opacifiers, binders, thickeners, buffering agents, pH regulators, complexing and sequestering agents, viscosity control agents, emulsifiers, emulsion stabilizers, film formers, solubilizers, antistats, electrolytes, foam stabilizers, propellants, peeling substances (abrasives, e.g. polymer beads or powders made of polyethylene, polypropylene etc. inorganic oxides, silicates etc.), fats, oils, waxes and/or silicone derivatives. If particles are present, these auxiliaries can be incorporated in the particles and/or in the medium in which the particles are suspended.

The total amount of auxiliaries in a gel or composition according to the present invention will usually be from about 0.001% to about 20% by weight, e.g., from about 0.01% to about 15% by weight, or from about 0.1% to about 10% by weight, based on the total weight of the composition.

Of the large number of organic solvents which are conventionally used in cosmetics, ethanol is the most prevalent. Other solvents which are conventionally used in cosmetics include polyhydric alcohols such as glycerol, butylene glycol and 2-methylpentane-2,4-diol, 2-ethoxyethanol, 2-butoxyethanol, aliphatic and aromatic hydrocarbons, ethyl acetate, methyl acetate, isobutyl acetate, isopropyl acetate, propyl acetate, phenyl acetate, ethylene carbonate, propylene carbonate, butanone, and benzaldehyde.

Non-limiting examples of antioxidants which may be incorporated into the gels and compositions of the present invention include amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes (e.g. α-carotene, β-carotene, lycopene) and derivatives thereof, chlorogenic acid and derivatives thereof, lipoic acid and derivatives thereof (e.g. dihydrolipoic acid), aurothioglucose, propylthiouracil and other thiols (e.g. thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (e.g. buthionine sulfoximine, homocysteine sulfoximine, buthionine sulfones, penta-, hexa-, heptathionine sulfoximine) in very low tolerated doses (e.g. pmol to umol/kg), also (metal) chelating agents (e.g. α-hydroxy fatty acids, palmitic acid, phytic acid, lactoferrin), α-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. γ-linolenic acid, linoleic acid, oleic acid), folic acid and derivatives thereof, vitamin C and derivatives (e.g. ascorbyl palmitate, Mg ascorbyl phosphate, ascorbyl acetate), tocopherols and derivatives (e.g. vitamin E acetate), vitamin A and derivatives (vitamin A palmitate) and coniferyl benzoate of benzoin resin, rutinic acid and derivates thereof, α-glycosylrutin, ferulic acid, furfurylideneglucitol, carnosine, butylhydroxytoluene, butylhydroxyanisole, nordihydroguaiacic acid, nordihydroguaiaretic acid, trihydroxybutyrophenone, uric acid and derivatives thereof, mannose and derivatives thereof, selenium and derivatives thereof (e.g. selenomethionine), stilbenes and derivatives thereof (e.g. stilbene oxide, trans-stilbene oxide) and the derivatives (salts, esters, ethers, sugars, nucleotides, nucleosides, peptides and lipids) of these specified active ingredients.

Non-limiting examples of advantageous active ingredients for incorporation into the gels and compositions of the present invention include natural active ingredients and/or derivatives thereof, such as, for example, alpha-lipoic acid, phytoene, D-biotin, coenzyme Q10, alpha-glucosylrutin, carnitine, carnosine, natural and/or synthetic isoflavonoids, creatin, fumaric esters, ectoin and derivatives thereof, taurine and β-alanine.

Gels and compositions according to the present invention which comprise, for example, known antiwrinkle active ingredients, such as flavone glycosides (in particular α-glycosylrutin), coenzyme Q10, vitamin E and/or derivatives and the like are particularly advantageously suitable for the prophylaxis and treatment of cosmetic or dermatological changes in the skin, as arise, for example, during skin ageing (such as, for example, dryness, roughness and formation of dryness wrinkles, itching, reduced regreasing (e.g. after washing), visible vascular dilations (teleangiectases, couperosis), flaccidity and formation of lines and wrinkles, local hyperpigmentation, hypopigmentation and incorrect pigmentation (e.g. age spots), increased susceptibility to mechanical stress (e.g. cracking) and the like). In addition, they are advantageously suitable for combating the appearance of dry and/or rough skin.

Other pharmaceutically or dermatologically effective substances, such as, for example, substances which calm and care for the skin, can also be incorporated into the gels and compositions of the present invention. Non-limiting examples thereof include panthenol, allantoin, tannin, antihistamines (e.g. loratadine, cetirizine, dimethindene, clemastine, capsaicin, H₁ antagonists, tannin preparations), local anaesthetics, opiate antagonists (e.g. naltrexone, naloxone), antiphlogistics, glucocorticoids (e.g. hydrocortisone, tacrolimus, cyclosporin A) and plant active ingredients, such as azulene and bisabolol, glycyrrhizin, hamamelin and plant extracts, such as camomile, aloe vera, hamamelis, liquorice. The vitamin D₃ analogues tacalcitol, calcipotriol, colecalciferol and calcitrol (vitamin D₃) and/or fumaric esters can also be advantageously incorporated into the gels and/or compositions of the present invention.

The gels and compositions of the present invention are also suitable for incorporating therein active ingredients for aiding the skin functions in dry skin such as, for example, vitamin C, biotin, carnitine, creatine, propionic acid, green tea extracts, eucalyptus oil, urea and mineral salts such as, for example, NaCl, sea minerals, and osmolytes such as, for example, taurine, inositol, betaine, quaternary ammonium compounds. In a similar way, the incorporation of active ingredients for alleviating or positively influencing irritative skin conditions, whether for sensitive skin in general or for skin irritated by noxae (UV light, chemicals) may be advantageous. In this regard, mention may be made of active ingredients such as sericosides, various extracts of licorice, licochalcones, in particular licochalcone A, silymarin, silyphos, dexpanthenol, inhibitors of prostaglandin metabolism, in particular of cyclooxygenase and of leukotriene metabolism, in particular of 5-lipoxygenase, but also of the 5-lipoxygenase inhibitor protein, FLAP. The incorporation of pigmentation modulators may also be advantageous. In this regard, mention may be made of active ingredients which reduce the pigmentation of the skin and thus lead to a cosmetically desired lightening of the skin, reduce the appearance of age spots and/or lighten existing age spots. Non-limiting examples of corresponding substances include tyrosine sulfate, dioic acid (8-hexadecene-1,16-dicarboxylic acid), and lipoic acid and liponamide, various extracts of licorice, kojic acid, hydroquinone, arbutin, alpha-arbutin, deoxyarbutin, bearberry (Uvae ursi), ursolic acid, ascorbic acid, green tea extracts, aminoguanidine, pyridoxamine.

The gels and compositions of the present invention further may comprise active ingredients which bring about an increased or more rapid tanning of the skin, be it with or without the effect of UV light, such as, e.g., Advanced Glycation Endproducts (AGE), lipofuscins, nucleic acid oligonucleotides, purines and pyrimidines, NO-releasing substances.

Non-limiting examples of advantageous moisturizers and humectants for use in the gels and compositions of the present invention include sorbitol, mannitol, glycerol, lactic acid and/or lactates, in particular sodium lactate, butylene glycol, propylene glycol, biosaccaride gum-1, glycine soya, ethylhexyloxy glycerol, pyrrolidone carboxylic acid and urea. In addition, it may be advantageous to use polymeric moisturizers from the group of water-soluble and/or water-swellable and/or water-gellable polysaccharides. Of particular advantage are, for example, hyaluronic acid, chitosan and/or a fucose-rich polysaccharide, which is filed in Chemical Abstracts under the registry number 178463-23-5 and is available, for example, under the name Fucogel®1000 from SOLABIA S.A.

The gels and compositions of the present invention may also comprise fillers which, for example, further improve the sensory and cosmetic properties thereof and bring about or enhance a velvety or silky feel on the skin. Advantageous fillers for the purposes of the present invention are starch and starch derivatives (such as, for example, tapioca starch, distarch phosphate, aluminum or sodium starch octenylsuccinate and the like), pigments which have neither primarily a UV filter effect nor a colouring effect (such as, for example, boron nitride etc.) and/or Aerosils^{®} (CAS No. 7631-86-9).

UV filter substances which may be incorporated into the gels and compositions of the present invention include those which are conventionally used in cosmetic or dermatological preparations.

Althought the gels of the present invention may comprise a variety of materials in addition to those which are necessary for the formation of the gel, i.e., compounds (a) and (b) as well as water, the gels of the present invention may also consist or essentially consist of (a), (b) and (c). Moreover, even if other materials are present, it may be desirable under certain circumstances to provide gels (and compositions) of the present invention which are (substantially) free of one or more of the following materials:
- organic gel formers and in particular, purely organic gel formers such as, e.g., dibenzylidene monosorbitol acetal;
- organic thickeners and/or crosslinking agents (e.g., cellulose and derivatives thereof, polysaccharides, etc.);
- zirconium compounds;
- fats and/or oils and/or waxes;
- silicon-containing organic compounds (e.g., silicone oils and silicone emulsifiers);
- alcohols (for example, monohydric and polyhydric alcohols such as, e.g., ethanol, ethylene glycol, propylene glycol, glycerin, PEGs).

For example, if a gel of the present invention is to be used for, e.g., the production of a (clear) glass, the substantial absence of any organic materials may be desirable. In this regard, it is pointed out that while the gels and compositions of the present invention have been described herein predominantly for use in cosmetic applications, the gels of the present invention can be employed in all fields in which (predominantly or purely) inorganic gels are useful. Merely by way of example, a gel of the present invention may be used as a gel for physical examinations by ultrasonic radiation or as a vehicle for transporting (administering) pharmaceutically active ingredients to or through the skin. It may also be further processed by heating it to elevated temperatures until it forms a (preferably substantially transparent and/or colorless) glass. This glass may have applications in the field of optics, illumination, signaling, etc. One of ordinary skill in the art will be aware of many other applications in which the use of a gel according to the present invention would be advantageous.

To store and apply the gels and compositions of the present invention, conventional containers and devices, e.g., containers and devices used for cosmetic or dermatological compositions can be used. For example, if the compositions are present in the form of a gel, a stick or a viscous liquid, stick dispensers, gel dispensers, tubes and roll-ons are particularly preferred. If the compositions are transparent or translucent and/or comprise suspended particles, preference is given to translucent, clear containers through which the compositions are visible.

Especially in cases where the preparation comprises suspended particles, particularly advantageous applicators include those which are described in DE 100 47 448, the entire disclosure whereof is expressly incorporated by reference herein.

The (substantially solid) composition of the present invention which comprises components (a) and (b) as set forth above for the gel of the present invention and contains not more than about 5 % by weight of water, based on the total weight of the composition (including any water that may be present in components (a) and/or (b) as complex/crystal water) differs from the gel of the present invention (and the compositions comprising the gel) essentially only in that it is not present in the form of a gel and comprises (considerably) less water, if any, than the gel. Accordingly, what has been set forth above with respect to the gel of the present invention applies *mutatis mutandis* also to the composition. Preferably, the water content of this composition is not higher than about 2 % by weight, e.g., not higher than about 1 % by weight, not higher than about 0.5 % by weight, or not higher than about 0.1 % by weight. More preferably, the composition is substantially anhydrous.

It has unexpectedly been found that when applied to (sweaty) skin the composition forms a gel with the water from the sweat, which gel is capable of stopping up pores of the skin and thus reduces or prevents further sweating (i.e., the composition has *in situ* antiperspirant activity). Accordingly, the composition of the present invention may be used for the same purposes as the gel of the present invention.

### EXAMPLES

The following examples illustrate the present invention without limiting the scope thereof in any way. Unless stated otherwise, all percentages given in the following are by weight. The Aluminum Chlorohydrate used in the examples is a commercial product in the form of a 50 % by weight aqueous solution having an atomic ratio Al:Cl of about 1.9-2.1:1 (available under the tradenames Aloxioll L, Chlorhydrol 50, AL 123).

### Example 1

| Aqueous Phase | |
|---|---|
| MgCO₃ | 0.9 % |
| Water | 73.3 % |

| Perfume Phase | |
|---|---|
| Perfume | 0.8 % |
| PEG-40 Hydrogenated Castor Oil | 5.0 % |

| ACH Phase | |
|---|---|
| Aluminum Chlorohydrate (50 %, remainder water) | 20.0 % |

### Example 2

| Aqueous Phase | |
|---|---|
| CaCO₃ | 0.95 % |
| Water | 73.25 % |

| Perfume Phase | |
|---|---|
| Perfume | 0.8 % |
| PEG-40 Hydrogenated Castor Oil | 5.0 % |

| ACH Phase | |
|---|---|
| Aluminum Chlorohydrate (50 %, remainder water) | 20.0 % |

### Example 3

| Aqueous Phase | |
|---|---|
| Na₂CO₃ | 1.1 % |
| Water | 73.1 % |

| Perfume Phase | |
|---|---|
| Perfume | 0.8 % |
| PEG-40 Hydrogenated Castor Oil | 5.0 % |

| ACH Phase | |
|---|---|
| Aluminum Chlorohydrate (50 %, remainder water) | 20.0 % |

### General Procedure for Production of Gel

### 1. Laboratory Scale (0.2 - 2.0 kg)

The process is carried out at room temperature. For preparing the aqueous phase the carbonate and the water are stirred in a beaker by means of a magnetic stirrer for about 15 minutes at 200 - 400 rpm (depending on the size of the batch) to ensure a uniform distribution and a complete wetting of the carbonate particles. In another beaker the perfume phase is prepared by stirring PEG-40 hydrogenated castor oil and the perfume by means of a magnetic stirrer for about 15 minutes at 100 - 200 rpm (depending on the size of the batch) without incorporating too much air into the mixture. Thereafter the aluminum chlorohydrate phase is added to the perfume phase and stirring is continued for another two minutes. The perfume phase with the added aluminum chlorohydrate phase is then added to the aqueous phase and the resultant mixture is stirred for about 20 minutes until the carbonate has completely dissolved and gel formation has started. The resultant transparent gel should thereafter be filled into appropriate containers within about 30 minutes.

### 2. Pilot Plant (12 kg)

The procedure is carried out at room temperature. To prepare the aqueous phase the carbonate is suspended in the water by means of a Becomix 15 CD mixer for about 15 minutes at 1 m/s to ensure a uniform distribution and a complete wetting of the carbonate particles. The perfume phase is prepared in a beaker by stirring PEG-40 hydrogenated castor oil and the perfume by means of a magnetic stirrer for about 15 minutes. Thereafter the perfume phase and then the aluminum chlorohydrate phase are sucked into the mixer and the resultant mixture is stirred at 1 m/s for about 30 minutes until the carbonate has completely dissolved and gel formation has started. The resultant transparent gel should thereafter be filled into appropriate containers within about 2 hours. This procedure can also be used for a production of the gel on an industrial scale (e.g., batch size 1,000 - 5,000 kg).

Examples of solubilizers which can replace the PEG-40 hydrogenated castor oil in the above examples include:
Coceth-7 + PPG-1-PEG-9 Lauryl Glycol Ether + PEG-40 Hydrogenated Castor Oil (available under the trade name Emulgin HPS) at a concentration of about 3-5 % (particularly preferred about 3 %);
Isosteareth 20 at a concentration of about 5 % (in this case the formation of the gel will be accelerated, with gel formation being substantially complete already after about 2 minutes);
PEG Hydrogenated Castor Oil containing about 10 % of water at a concentration of about 5 % (in this case some of the water is already "pre-emulsified", which may be advantageous).

### Determination of Apparent Yield Point of the Gel of Example 1

The apparent yield point of the gel of Example 1 was determined by means of a plate-plate measuring system (SR-2000, Rheometric Scientific, Munich, Germany; width of measuring gap 1 mm, diameter 25 mm). At a temperature of 25°C the shear stress was steadily increased from 0 Pa at a rate of 40 Pa/min. The shear stress at which the viscosity reached a maximum was taken as the apparent yield point of the gel.

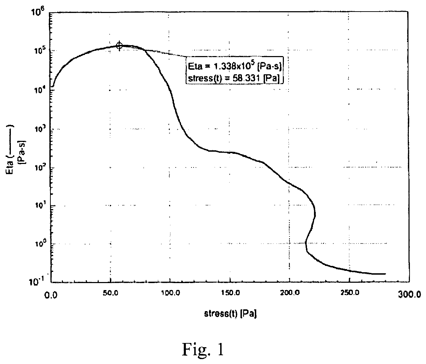

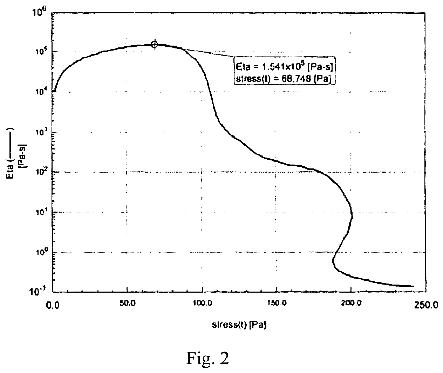

The obtained results are set forth in the table below, which shows the results for gels with two different perfumes (perfume A and perfume B). The viscosities of the gels as a factor of the applied shear stress are graphically represented in Figures 1 and 2.

| Sample | Yield Point @ 25 °C | |
|---|---|---|
| | Shear Stress τₘₐₓ [Pa] | Viscosity ηₘₐₓ |
| Example 1a 0.8 % Perfume A | 58.3 | 133,800 |
| Example 1b 0.8 % Perfume B | 68.8 | 154,100 |

The graphs of Figures 1 and 2 show an unexpectedly broad yield point up to about 100 Pa. This is the force which is needed to make the gel flow and indicates a dense structure within the gel. On the other hand, at a shear stress below the maximum shear stress a permanent deformation of the gel can be observed, which indicates a stabilization against settling of particles such as beads which may be incorporated into the gel, for example, in order to provide a modified release of active ingredient(s) or for decorative purposes. During the measurement the shear stress is steadily increased. It is surprising that in contrast to conventional gels the viscosity of the gels of the present invention decreases in a step-wise fashion. This is assumed to be due to the presence of a stable, coherent gel skeleton. The dense structure is indicative of a high storage stability. The gels start to flow only at a shear stress of about 250 Pa. Conventional gels show a narrow range of the yield point without a step-wise decrease of the viscosity.

Perfumes A and B give rise to a small difference in the shear stress at which the yield point is determined. Differences of >5 % are considered to be significant. Accordingly, the rheological properties of the gels of the present invention may surprisingly be adjusted also by the type of the perfume incorporated therein.

### Determination of the Influence of the pH of the Aqueous Phase on Gelation Time and Transparency of Gel Product

Using the composition of Example 1, the influence of the pH of the aqueous phase on the time needed for the formation of the gel (at room temperature) and the transparency of the resultant gel was determined. The obtained results are set forth in the following table.

| **pH of Aqueous Phase** | **3.0** | **7.0** | **12.0** |
|---|---|---|---|
| Time [min] to formation of gel after addition of ACH | 17 min | 6 min | 1 min 30 s |
| Transparency of gel [after 24 h] | yes | yes | no |

As can be taken from the above results, at pH 7 the gel forms within 6 minutes. At a pH of 3 the required time increases to 17 minutes, with a transparent gel still being formed. At a pH of 12 the time required for gel formation decreases to 1.5 minutes but the obtained gel is no longer transparent. It is surprising that over the broad pH range of from 3 to 12 a stable gel skeleton is formed.

### Determination of the Influence of the Temperature of the Aqueous Phase on Gelation Time and Transparency of Gel Product

Using the composition of Example 1, the influence of the temperature of the aqueous phase (pH = 5.8) on the time needed for the formation of the gel and the transparency of the resultant gel was determined. The obtained results are set forth in the following table.

| **Temperature of Aqueous Phase [°C]** | **RT** | **+40°C** | **+80°C** |
|---|---|---|---|
| Time [min] to formation of gel after addition of ACH | 6 min | 22 min | 40 min |
| Transparency of gel [after 24 h] | yes | no | no |
| Generation of gas during formation of gel | no | yes | yes |

As can be seen from the above results, an increase of the temperature of the aqueous phase from room temperature to 40°C or 80°C increases the time that is required for the formation of the gel from 6 minutes to 22 minutes or 40 minutes respectively. Moreover, a transparent gel is formed only at room temperature whereas a strong but opaque gel is formed at the higher temperatures. Also, a strong generation of gas is observed at 40°C and 80°C, which may be due to the formation of carbon dioxide.

### Example 4

| Aqueous Phrase | |
|---|---|
| MgCO₃ | 0.9 % |
| Water | 79.1 % |

| ACH Phrase | |
|---|---|
| Aluminum Chlorohydrate (50 %, remainder water) | 20.0 % |

### Example 5

| Aqueous Phase | |
|---|---|
| CaCO₃ | 0.95 % |
| Water | 79.05 % |

| ACH Phase | |
|---|---|
| Aluminum Chlorohydrate (50 %, remainder water) | 20.0 % |

### Example 6

| Aqueous Phase | |
|---|---|
| Na₂CO₃ | 1.1 % |
| Water | 78.9 % |

| ACH Phase | |
|---|---|
| Aluminum Chlorohydrate (50 %, remainder water) | 20.0 % |

### Example 7

| Aqueous Phase | |
|---|---|
| MgCO₃ | 0.95 % |
| Water | 79.05 % |

| ACH Phase | |
|---|---|
| Aluminum Chlorohydrate (50 %, remainder water) | 20.0 % |

### Example 8

| Aqueous Phase | |
|---|---|
| CaCO₃ | 1.00 % |
| Water | 79.00 % |

| ACH Phase | |
|---|---|
| Aluminum Chlorohydrate (50 %, remainder water) | 20.0 % |

### General Procedure for Production of Gel

The procedure is carried out analogously to what is described above for Examples 1-3. The absence of the perfume phase and in particular, of the solubilizer (PEG-40 hydrogenated castor oil) does not significantly affect the physical properties of gel produced but results in a lengthening of the period that is required for gel formation by about 30 %.

### Determination of Time Dependence of Dynamic Viscosity (Examples 4 and 5)

The measurement of the dynamic viscosity was carried out at room temperature by means of a rotary viscometer (VT02 measuring system Rheomat 123, proRheo, Althengstett, Germany). The sample was placed within the annular gap between two concentric cylinders, with the inner cylinder rotating according to the Searle principle. The inherent viscosity gave rise to a torque at the inner cylinder. The size of the torque was measured by means of the twisting angle of a spring subjected to torsion. After each shearing operation the viscosity was recorded for 30 s. The obtained values allow an assessment of the flow properties of the systems. The obtained results are listed in the table below.

| | **Viscosity (in MPa.s) at RT** | |
|---|---|---|
| t [min] (time after addition of ACH phase) | Example 4 (0.9 % MgCO₃) | Example 5 (0.95 % CaCO₃) |
| 1 | Start of gel formation | Start of gel formation |
| 2 | | |
| 3 | | |
| 4 | 1300 | |
| 5 | 3050 | |
| 6 | 3950 | |
| 7 | 4700 | |
| 8 | 5600 | 1850 |
| 9 | 6300 | 1950 |
| 10 | 7250 | 2000 |
| 11 | 7950 | 2150 |
| 12 | 9250 | 2350 |
| 13 | 9450 | 2350 |
| 14 | not measurable | 2450 |
| 15 | not measurable | 2600 |
| 20 | not measurable | 4250 |

The results set forth above show that gel formation takes place over a broad range of viscosities. Surprisingly, the gel shows a high viscosity already after a few minutes - in contrast to gels based on celluloses or starches. This permits a preparation of the gels within short periods (no time required for swelling of the gel) and without heating.

**Improving Smoothness of Gel by Addition of Citric Acid**

| | | **Example No.** | | | | | |
|---|---|---|---|---|---|---|---|
| **Phase** | **Components** | **9** | **10** | **11** | **12** | **13** | **14** |
| A | Propylene Glycol | 3.000 | 3.000 | 3.000 | 3.000 | 3.000 | 3.000 |
| | Water | 79,250 | 79.150 | 79.100 | 79.050 | 79.000 | 78.750 |
| | Citric Acid | | 0.100 | 0.150 | 0.200 | 0.250 | 0.500 |
| | Magnesium Carbonate | 0.950 | 0.950 | 0.950 | 0.950 | 0.950 | 0.950 |
| B | PEG-40 Hydrogenated Castor Oil | 5.000 | 5.000 | 5.000 | 5.000 | 5.000 | 5.000 |
| | PEG/PPG-20/20 Dimethicone | 1.000 | 1.000 | 1.000 | 1.000 | 1.000 | 1.000 |
| | Parfum | 0.800 | 0.800 | 0.800 | 0.800 | 0.800 | 0.800 |
| C | Aluminum Chlorohydrate (100 % active) | 10.000 | 10.000 | 10.000 | 10.000 | 10.000 | 10.000 |
| Smoothness | | 1 | 2 | 3 | 3 | 4 | 5 |

Procedure (room temperature): Disperse Phase A for 15 minutes (16,000 rpm) and stir Phase B for 15 minutes (magnetic stirrer). Combine Phases A and B and stir for 15 minutes (magnetic stirrer). Then add Phase C and stir (magnetic stirrer) until the mixture begins to form a gel.

Smoothness : by organoleptic evaluation (1 - 5); most preferred: 3, 4
1: very hard surface, rigid gel
2: hard surface, rigid gel
3: smooth surface, rigid gel
4: smooth surface, smooth gel
5: very soft gel

**Freeze Stabilization of Gel by Addition of Propylene Glycol**

| | | **Example No.** | | |
|---|---|---|---|---|
| **Phase** | **Components** | **15** | **16** | **17** |
| A | Propylene Glycol | 4.000 | 6.000 | 8.000 |
| | Aqua | 78.075 | 76.075 | 74.075 |
| | Citric Acid | 0.175 | 0.175 | 0.175 |
| | Magnesium Carbonate | 0.950 | 0.950 | 0.950 |
| B | PEG-40 Hydrogenated Castor Oil | 5.000 | 5.000 | 5.000 |
| | PEG/PPG-20/20 Dimethicone | 1.000 | 1.000 | 1.000 |
| | Parfum | 0.800 | 0.80 | 0.80 |
| C | Aluminum Chlorohydrate (100 % active) | 10.000 | 10.0000 | 10.000 |
| cycle test (6d: +40°C/-10°C) | | not stable | not stable | stable |

Procedure (room temperature): Disperse Phase A for 15 minutes (16,000 rpm) and stir Phase B for 15 minutes (magnetic stirrer). Combine Phases A and B and stir for 15 minutes (magnetic stirrer). Then add Phase C and stir (magnetic stirrer) until the mixture begins to form a gel.

**Freeze Stabilization of Gel by Addition of Ethanol**

| | | **Example No.** | | | |
|---|---|---|---|---|---|
| **Phase** | **Components** | **18** | **19** | **20** | **21** |
| A | Propylene Glycol | 6.000 | 6.000 | 6.000 | 6.000 |
| | Aqua | 75.075 | 74.075 | 73.075 | 72.075 |
| | Citric Acid | 0.175 | 0.175 | 0.175 | 0.175 |
| | Magnesium Carbonate | 0.950 | 0.950 | 0.950 | 0.950 |
| B | PEG-40 Hydrogenated Castor Oil | 5.000 | 5.000 | 5.000 | 5.000 |
| | PEG/PPG-20/20 Dimethicone | 1.000 | 1.000 | 1.000 | 1.000 |
| | Parfum | 0.800 | 0.800 | 0.800 | 0.800 |
| C | Alcohol Denat, | 1.000 | 2.000 | 3.000 | 4.000 |
| D | Aluminum Chlorohydrate (100 % active) | 10.000 | 10.000 | 10.000 | 10.000 |
| cycle test (6d: +40°C/ -10°C) | | not stable | not stable | stable | stable |

Procedure (room temperature): Disperse Phase A for 15 minutes (16,000 rpm) and stir Phase B for 15 minutes (magnetic stirrer). Combine Phases A and B and stir for 15 minutes (magnetic stirrer). Add Phase C and stir (magnetic stirrer) for 15 minutes. Then add Phase D and stir until the mixture begins to form a gel.

It is noted that the foregoing examples have been provided merely for the purpose of explanation and are in no way to be construed as limiting of the present invention. While the present invention has been described with reference to exemplary embodiments, it is understood that the words which have been used herein are words of description and illustration, rather than words of limitation. Changes may be made, within the purview of the appended claims, as presently stated and as amended, without departing from the scope and spirit of the present invention in its aspects. Although the present invention has been described herein with reference to particular means, materials and embodiments, the present invention is not intended to be limited to the particulars disclosed herein; rather, the present invention extends to all functionally equivalent structures, methods and uses, such as are within the scope of the appended claims.

## Claims

1. An inorganic gel, **characterized in that** the gel is based on (a) one or more aluminum compounds, (b) one or more compounds selected from inorganic carbonate compounds and inorganic bicarbonate compounds and (c) water, wherein a weight ratio (a) : (b) is from 20 : 1 to 2 : 1.

2. The gel of claim 1, wherein the gel has a pH of from 4 to 6.

3. The gel of any one of claims 1 to 2, wherein the gel is transparent

4. The gel of any one of claims 1 to 3, wherein (a) comprises at least one of aluminum chlorohydrate, activated aluminum chlorohydrate and aluminum sesquichlorohydrate.

5. The gel of any one of claims 1 to 4, wherein (b) comprises at least one compound selected from carbonates and bicarbonates of alkali metals, alkaline earth metals, zinc and ammonium.

6. The gel of any one of claims 1 to 5, wherein (b) comprises MgCO₃.

7. The gel of any one of claims 1 to 6, wherein (b) is employed in a concentration of from 0.2 % to about 5 % by weight, based on a total weight of (a) plus (b) plus (c).

8. The gel of any one of claims 1 to 7, wherein the gel further comprises a perfume and a solubilizer for the perfume.

9. The gel of claim 8, wherein the solubilizer comprises at least one of a polyethoxylated fatty acid mono-, di- or triglyceride and a polyethoxylated fatty alcohol.

10. The gel of any one of claims 1 to 9, wherein the gel further comprises a plurality of particles suspended therein.

11. The gel of any one of claims 1 to 10, wherein the gel is free of organic gel formers.

12. The gel of any one of claims to 1 to 7, wherein the gel is free of organic substances.

13. A gel with antiperspirant activity, wherein the gel is transparent, has a pH of from 4 to 6, and is obtainable by a process which comprises contacting, at atmospheric pressure and at a temperature of from 10°C to 40°C, (a) from 9% to 11 % by weight of at least one of aluminum chlorohydrate and activated aluminum chlorohydrate, and (b) from 0.9 % to 1.2 % by weight of at least one of Na₂CO₃, K₂CO₃, MgCO₃, CaCO₃, (NH₄)₂CO₃, ZnCO₃, NaHCO₃, and KHCO₃ in the presence of (c) at least 70 % by weight of water, all weight percentages being based on a total weight of (a) plus (b) plus (c).

14. A cosmetic or dermatological composition, wherein the composition comprises the gel of any one of claims 1 to 13.

## Patentansprüche

1. Anorganisches Gel, **dadurch gekennzeichnet, dass** das Gel auf (a) einer oder mehreren Aluminiumverbindungen, (b) einer oder mehreren Verbindungen, die aus anorganischen Carbonatverbindungen und anorganischen Hydrogencarbonatverbindungen ausgewählt sind, und (c) Wasser basiert, wobei das Gewichtsverhältnis (a) : (b) 20:1 bis 2:1 beträgt.

2. Gel nach Anspruch 1, wobei das Gel einen pH-Wert von 4 bis 6 aufweist.

3. Gel nach einem der Ansprüche 1 bis 2, wobei das Gel transparent ist.

4. Gel nach einem der Ansprüche 1 bis 3, wobei (a) Aluminiumchlorhydrat, aktiviertes Aluminiumchlorhydrat und/oder Aluminiumsesquichlorhydrat umfasst.

5. Gel nach einem der Ansprüche 1 bis 4, wobei (b) mindestens eine Verbindung umfasst, die aus Carbonaten und Hydrogencarbonaten von Alkalimetallen, Erdalkalimetallen, Zink und Ammonium ausgewählt ist.

6. Gel nach einem der Ansprüche 1 bis 5, wobei (b) MgCO₃ umfasst.

7. Gel nach einem der Ansprüche 1 bis 6, wobei (b) in einer Konzentration von 0,2 bis etwa 5 Gew.-%, bezogen auf das Gesamtgewicht von (a) plus (b) plus (c), eingesetzt wird.

8. Gel nach einem der Ansprüche 1 bis 7, wobei das Gel ferner ein Parfüm und einen Löslichkeitsvermittler für das Parfüm umfasst.

9. Gel nach Anspruch 8, wobei der Löslichkeitsvermittler ein polyethoxyliertes Fettsäuremono-, -di- oder -triglycerid und/oder einen polyethoxylierten Fettalkohol umfasst.

10. Gel nach einem der Ansprüche 1 bis 9, wobei das Gel ferner eine Vielzahl von darin suspendierten Teilchen umfasst.

11. Gel nach einem der Ansprüche 1 bis 10, wobei das Gel frei von organischen Gelbildnern ist.

12. Gel nach einem der Ansprüche 1 bis 7, wobei das Gel frei von organischen Substanzen ist.

13. Gel mit Antitranspirantwirkung, wobei das Gel transparent ist, einen pH-Wert von 4 bis 6 aufweist und durch ein Verfahren erhältlich ist, bei dem man bei Normaldruck und bei einer Temperatur von 10°C bis 40°C (a) 9 bis 11 Gew.-% Aluminiumchlorhydrat und/oder aktiviertes Aluminiumchlorhydrat und (b) 0,9 bis 1,2 Gew.-% Na₂CO₃, K₂CO₃, MgCO₃, CaCO₃, (NH₄)₂CO₃, ZnCO₃, NaHCO₃ und/oder KHCO₃ in Gegenwart von (c) mindestens 70 Gew.-% Wasser in Berührung bringt, wobei sich alle Gewichtsprozentangaben auf das Gesamtgewicht von (a) plus (b) plus (c) beziehen.

14. Kosmetische oder dermatologische Zusammensetzung, wobei die Zusammensetzung das Gel nach einem der Ansprüche 1 bis 13 umfasst.

## Revendications

1. Gel inorganique, **caractérisé en ce que** le gel est à base (a) d'un ou plusieurs composés d'aluminium, (b) d'un ou plusieurs composés choisis parmi les composés de carbonate inorganiques et les composés de bicarbonate inorganiques, et (c) d'eau, où un rapport pondéral (a) : (b) va de 20:1 à 2:1.

2. Gel selon la revendication 1, **caractérisé en ce que** le gel possède un pH allant de 4 à 6.

3. Gel selon l'une quelconque des revendications 1 à 2, **caractérisé en ce que** le gel est transparent.

4. Gel selon l'une quelconque des revendications 1 à 3, **caractérisé en ce que** (a) comprend au moins un parmi le chlorhydrate d'aluminium, le chlorhydrate d'aluminium activé et le sesquichlorohydrate d'aluminium.

5. Gel selon l'une quelconque des revendications 1 à 4, **caractérisé en ce que** (b) comprend au moins un composé choisi parmi les carbonates et les bicarbonates de métaux alcalins, de métaux alcalino-terreux, de zinc et d'ammonium.

6. Gel selon l'une quelconque des revendications 1 à 5, **caractérisé en ce que** (b) comprend du MgCO₃.

7. Gel selon l'une quelconque des revendications 1 à 6, **caractérisé en ce que** (b) est employé selon une concentration allant de 0,2% à environ 5% en poids, sur la base du poids total de (a) plus (b) plus (c).

8. Gel selon l'une quelconque des revendications 1 à 7, **caractérisé en ce que** le gel comprend en outre un parfum et un agent solubilisant pour le parfum.

9. Gel selon la revendication 8, **caractérisé en ce que** l'agent solubilisant comprend au moins un parmi un mono-, di- ou triglycéride d'acide gras polyéthoxylé et un alcool gras polyéthoxylé.

10. Gel selon l'une quelconque des revendications 1 à 9, **caractérisé en ce que** le gel comprend en outre une pluralité de particules y étant suspendues.

11. Gel selon l'une quelconque des revendications 1 à 10, **caractérisé en ce que** le gel est dépourvu d'agents gélifiants organiques.

12. Gel selon l'une quelconque des revendications 1 à 7, **caractérisé en ce que** le gel est dépourvu de substances organiques.

13. Gel ayant une activité antitranspirante, **caractérisé en ce que** le gel est transparent, possède un pH allant de 4 à 6, et peut être obtenu par un procédé comprenant la mise en contact, à pression atmosphérique et à une température allant de 10°C à 40°C, (a) de 9% à 11% en poids d'au moins un parmi le chlorhydrate d'aluminium et le chlorhydrate d'aluminium activé, et (b) de 0,9% à 1,2% en poids d'au moins un parmi Na₂CO₃, K₂CO₃, MgCO₃, CaCO₃, (NH₄)₂CO₃, 2nCO₃, NaHCO₃, et KHCO₃, en présence (c) d'au moins 70% en poids d'eau, tous les pourcentages pondéraux étant sur la base d'un poids total de (a) plus (b) plus (c) .

14. Composition cosmétique ou dermatologique, **caractérisée en ce que** la composition comprend le gel selon l'une quelconque des revendications 1 à 13.
